# EUROPEAN PATENT APPLICATION

(11) **EP 2 163 277 A2**
(43) Date of publication of application: **17.03.2010**
(21) Application number: 09252184.8
(22) Date of filing: 15.09.2009
(51) Int. Cl.: A61M 25/01, A61M 25/09

(54) **Torque device with side attachment**

(30) Priority: 15.09.2008 US 192200 P
(71) Applicant: Elcam Agricultural Cooperative Association Ltd., 13860 Kibbutz Bar-Am (IL)
(72) Inventor: Haskal, Ziv, Cockeysville, Maryland 21030 (US); Naftalovitz, Ziv, D.N. West Galilee 22845 (IL); Schlezinger, Amit, D.N. Merom Hagalil 13860 (IL); Slobitker, Leon, Karmiel (IL)
(74) Representative: White, Duncan Rohan

(57) **Abstract**

A device for manual manipulation of a guidewire (164) including a generally cylindrical, integrally formed, compressible element (104, 106) arranged along a longitudinal axis (102) and being compressible transversely to the longitudinal axis (102) and having at least fully compressed and partially compressed operative orientations, the element (104, 106) in the fully compressed operative orientation defining an axial slot adapted for slidable side mounting of the element (104, 106) onto a guidewire (164) and the element (104, 106) in the partially compressed operative orientation being adapted for tightly engaging a guidewire (164) in the slot such that axial and rotational movement of the element (104, 106) produces corresponding axial and rotational movement of the guidewire (164).

## Description

### FIELD OF THE INVENTION

The present invention relates generally to devices for manual manipulation of a guidewire in medical applications.

### BACKGROUND OF THE INVENTION

The following patents are believed to represent the current state of the art: U.S. Patents 7,186,224; 6,949,104; 6,916,293; 6,746,466; 6,533,772; 6,511,470; 6,485,466; 6,190,333; 6,059,484; 6,039,722; 5,851,189; 5,634,475; 6,507,300; 5,392,778; 5,219,332; 5,161,534; 5,137,517; 5,137,288; 4,957,117 and 4,726,117 and European Patent 0534747.

### SUMMARY OF THE INVENTION

The present invention seeks to provide an improved device for manual manipulation of a guidewire in medical applications.

There is thus provided in accordance with a preferred embodiment of the present invention a device for manual manipulation of a guidewire including a generally cylindrical, integrally formed, compressible element arranged along a longitudinal axis and being compressible transversely to the longitudinal axis and having at least fully compressed and partially compressed operative orientations, the element in the fully compressed operative orientation defining an axial slot adapted for slidable side mounting of the element onto a guidewire and the element in the partially compressed operative orientation being adapted for tightly engaging a guidewire in the slot such that axial and rotational movement of the element produces corresponding axial and rotational movement of the guidewire.

In accordance with a preferred embodiment of the present invention the element includes first and second tooth bearing side portions each having a plurality of mutually spaced teeth, the mutually spaced teeth each having forward and back portions separated by a gap.

Preferably, first and second end portions are integrally formed with the first and second tooth-bearing side portions, the first and second end portions defining respective first and second notches which are mutually aligned along the longitudinal axis.

In accordance with a preferred embodiment of the present invention the first and second tooth-bearing side portions are arranged so as to be displaceable towards each other in directions transverse to the longitudinal axis such that the element assumes the fully compressed operative orientation wherein the teeth of the first and second tooth-bearing side portions are interdigitated such that the gaps of the plurality of mutually spaced teeth on both of the first and second tooth-bearing side portions are aligned along the longitudinal axis to define the slot extending between the first and second notches along the longitudinal axis and displaceable away from each other in directions opposite to the directions transverse to the longitudinal axis when a guidewire is located in the slot, such that the element assumes the partially compressed operative orientation wherein the teeth of the first and second tooth-bearing side portions grip the guidewire along the longitudinal axis, whereby axial and rotational movement of the element is effective to produce corresponding axial and rotational movement of the guidewire.

Preferably, the first and second tooth-bearing side portions each include an outwardly facing back surface having an elongate rib and a pair of transverse end ribs and the first and second tooth-bearing side portions also include relatively flexible portions extending longitudinally outwards from each of the transverse end ribs and coupling the first and second tooth-bearing side portions to the first and second end portions.

Preferably, the first and second end portions each include two exterior side portions joined to the relatively flexible portions and being bent inwardly to define an axially facing opening having a v-shaped cross section.

Preferably, the first and second end portions are identical.

Preferably, the forward and back portions of the mutually spaced teeth are joined by a transverse bottom portion.

Preferably, the forward and back portions extend parallel to each other and perpendicular to the longitudinal axis.

Preferably, adjacent ones of the mutually spaced teeth are spaced by a separation, the separation being equal to a width of each of the mutually spaced teeth.

Preferably, the mutually spaced teeth of the first tooth-bearing side portion are offset in a direction along the longitudinal axis with respect to the mutually spaced teeth of the second tooth-bearing side portion by the width of each of the mutually spaced teeth, such that when the element is in the uncompressed orientation the forward portions of the mutually spaced teeth of the first and second tooth-bearing side portions are interdigitated along the longitudinal axis and when the element assumes the fully compressed operative orientation, the forward portions of the mutually spaced teeth are fully or nearly fully seated in the separations.

Preferably, the first and second tooth-bearing side portions are linearly offset mirror images of each other.

Additionally or alternatively, the element assumes an overall circular cross section when the element is in the fully compressed orientation.

Preferably, the element is integrally formed of an elastic material.

In accordance with another preferred embodiment of the present invention the guidewire may be axially and rotationally moved by a user without producing corresponding axial and rotational movement of the element, when the element is in the partially compressed operative orientation.

There is further provided in accordance with another preferred embodiment of the present invention a device for manual manipulation of a guidewire including a generally cylindrical, integrally formed, compressible element arranged along a longitudinal axis and being compressible transversely to the longitudinal axis and having first, second and third, respectively uncompressed, fully compressed and partially compressed operative orientations, the element in the fully compressed operative orientation defining an axial slot adapted for side mounting of the element onto a guidewire and the element in the partially compressed operative orientation being adapted for tightly engaging a guidewire in the slot such that axial and rotational movement of the element produces corresponding axial and rotational movement of the guidewire.

In accordance with a preferred embodiment of the present invention the element includes first and second tooth bearing side portions each having a plurality of mutually spaced teeth, the mutually spaced teeth each having forward and back portions separated by a gap.

Preferably, first and second end portions are integrally formed with the first and second tooth-bearing side portions, the first and second end portions defining respective first and second notches which are mutually aligned along the longitudinal axis.

In accordance with a preferred embodiment of the present invention the first and second tooth-bearing side portions are arranged so as to be displaceable towards each other in directions transverse to the longitudinal axis, such that the element assumes the fully compressed operative orientation wherein the teeth of the first and second tooth-bearing side portions are interdigitated such that the gaps of the plurality of mutually spaced teeth on both of the first and second tooth-bearing side portions are aligned along the longitudinal axis to define the slot extending between the first and second notches along the longitudinal axis and displaceable away from each other in directions opposite to the directions transverse to the longitudinal axis when a guidewire is located in the slot, such that the element assumes the partially compressed operative orientation wherein the teeth of the first and second tooth-bearing side portions grip the guidewire along the longitudinal axis, whereby axial and rotational movement of the element is effective to produce corresponding axial and rotational movement of the guidewire.

Preferably, the first and second tooth-bearing side portions each include an outwardly facing back surface having an elongate rib and a pair of transverse end ribs and the first and second tooth-bearing side portions also include relatively flexible portions extending longitudinally outwards from each of the transverse end ribs and coupling the first and second tooth-bearing side portions to the first and second end portions.

Preferably, the first and second end portions each include two exterior side portions joined to the relatively flexible portions and being bent inwardly to define an axially facing opening having a v-shaped cross section.

Preferably, the first and second end portions are identical.

Preferably, the forward and back portions of the mutually spaced teeth are joined by a transverse bottom portion.

Preferably, the forward and back portions extend parallel to each other and perpendicular to the longitudinal axis.

Preferably, adjacent ones of the mutually spaced teeth are spaced by a separation, the separation being equal to a width of each of the mutually spaced teeth.

Preferably, the mutually spaced teeth of the first tooth-bearing side portion are offset in a direction along the longitudinal axis with respect to the mutually spaced teeth of the second tooth-bearing side portion by the width of each of the mutually spaced teeth, such that when the element is in the uncompressed orientation the forward portions of the mutually spaced teeth of the first and second tooth-bearing side portions are interdigitated along the longitudinal axis and when the element assumes the fully compressed operative orientation, the forward portions of the mutually spaced teeth are fully or nearly fully seated in the separations.

Preferably, the first and second tooth-bearing side portions are linearly offset mirror images of each other.

Additionally or alternatively, the element assumes an overall circular cross section when the element is in the fully compressed orientation.

Preferably, the element is integrally formed of an elastic material.

In accordance with another preferred embodiment of the present invention the guidewire may be axially and rotationally moved by a user without producing corresponding axial and rotational movement of the element, when the element is in the partially compressed operative orientation.

There is additionally provided in accordance with yet another preferred embodiment of the present invention a device for manual rotation of a guidewire including first and second tooth-bearing side portions, the first and second tooth bearing side portions each having a plurality of mutually spaced teeth, the mutually spaced teeth each having forward and back portions separated by a gap and first and second end portions, integrally formed with the first and second tooth-bearing side portions, the first and second end portions defining respective first and second notches which are mutually aligned along a longitudinal guidewire-receiving axis, the first and second tooth-bearing side portions being arranged so as to be displaceable towards each other in directions transverse to the longitudinal guidewire-receiving axis such that the first and second tooth-bearing side portions assume a guidewire-receiving orientation wherein the teeth of the first and second tooth-bearing side portions are interdigitated such that the gaps of the plurality of mutually spaced teeth on both of the first and second tooth-bearing side portions are aligned along the longitudinal guidewire-receiving axis to define a guidewire-receiving slot extending between the first and second notches along the longitudinal guidewire-receiving axis and displaceable away from each other in directions opposite to the directions transverse to the longitudinal guidewire-receiving axis when a guidewire is located in the guidewire-receiving slot, such that the first and second tooth-bearing side portions assume a guidewire-gripping orientation wherein the teeth of the first and second tooth-bearing side portions grip the guidewire along the longitudinal guidewire-receiving axis, whereby manual rotation of the device about the longitudinal guidewire-receiving axis is effective to rotate the guidewire about the longitudinal guidewire-receiving axis.

Preferably, the first and second tooth-bearing side portions each include an outwardly facing back surface having an elongate rib and a pair of transverse end ribs and the first and second tooth-bearing side portions also include relatively flexible portions extending longitudinally outwards from each of the transverse end ribs and coupling the first and second tooth-bearing side portions to the first and second end portions.

Preferably, each of the first and second end portions includes two exterior side portions joined to the relatively flexible portions and being bent inwardly to define an axially facing opening having a v-shaped cross section.

Preferably, the first and second end portions are identical.

Preferably, the forward and back portions of the mutually spaced teeth are joined by a transverse bottom portion.

Preferably, the forward and back portions extend parallel to each other and perpendicular to the longitudinal guidewire-receiving axis.

Preferably, adjacent ones of the mutually spaced teeth are spaced by a separation, the separation being equal to a width of each of the mutually spaced teeth.

Preferably, the mutually spaced teeth of the first tooth-bearing side portion are offset in a direction along the longitudinal guidewire-receiving axis with respect to the mutually spaced teeth of the second tooth-bearing side portion by the width of each of the mutually spaced teeth, such that when the first and second tooth-bearing side portions are at rest, the forward portions of the mutually spaced teeth of the first and second tooth-bearing side portions are interdigitated along the longitudinal guidewire-receiving axis and when the first and second tooth-bearing side portions assume the guidewire-receiving orientation, the forward portions of the mutually spaced teeth are fully or nearly fully seated in the separations.

Preferably, the first and second tooth-bearing side portions are linearly offset mirror images of each other.

Additionally or alternatively, the device assumes an overall circular cross section when the first and second tooth-bearing side portions assume the guidewire-receiving orientation.

Preferably, the device is integrally formed of an elastic material and has a generally cylindrical configuration.

In accordance with another preferred embodiment of the present invention the guidewire may be axially and rotationally moved by a user without producing corresponding axial and rotational movement of the device, when the first and second tooth-bearing side portions are in the guidewire-gripping orientation.

### BRIEF DESCRIPTION OF THE DRAWINGS

The present invention will be understood and appreciated more fully from the following detailed description, taken in conjunction with the drawings in which:

Fig. 1 is a simplified composite, partially pictorial and partially sectional illustration of a device for manual manipulation of a guidewire in medical applications constructed and operative in accordance with a preferred embodiment of the present invention;

Figs. 2A, 2B & 2C are simplified respective pictorial, top view and sectional illustrations of the device of Fig. 1 in a first, uncompressed operative orientation;

Figs. 3A, 3B & 3C are simplified respective pictorial, top view and sectional illustrations of the device of Fig. 1 in a second, compressed operative orientation in which the device has a generally circular cross section;

Figs. 4A, 4B & 4C are simplified respective pictorial, top view and sectional illustrations of the device of Fig. 1 in the second, compressed operative orientation in which the device has a generally circular cross section, having a guidewire placed, but not retained, therein;

Figs. 5A, 5B & 5C are simplified respective pictorial, top view and sectional illustrations of the device of Fig. 1 in a third, partially compressed, operative orientation, having a guidewire retained therein;

Fig. 6A shows a user holding the device of Fig. 1 in its first operative orientation;

Fig. 6B shows a user holding the device of Fig. 1 in its second operative orientation;

Fig. 6C shows a user holding the device of Fig. 1 in its second operative orientation having a guidewire placed, but not retained, therein;

Fig. 6D shows a user holding the device of Fig. 1 in its third operative orientation, having a guidewire retained therein;

Fig. 6E illustrates manual axial movement of the device of Fig. 1 in its third operative orientation; and

Fig. 6F illustrates manual rotation of the device of Fig. 1 in its third operative orientation, through 90 degrees.

### DETAILED DESCRIPTION OF A PREFERRED EMBODIMENT

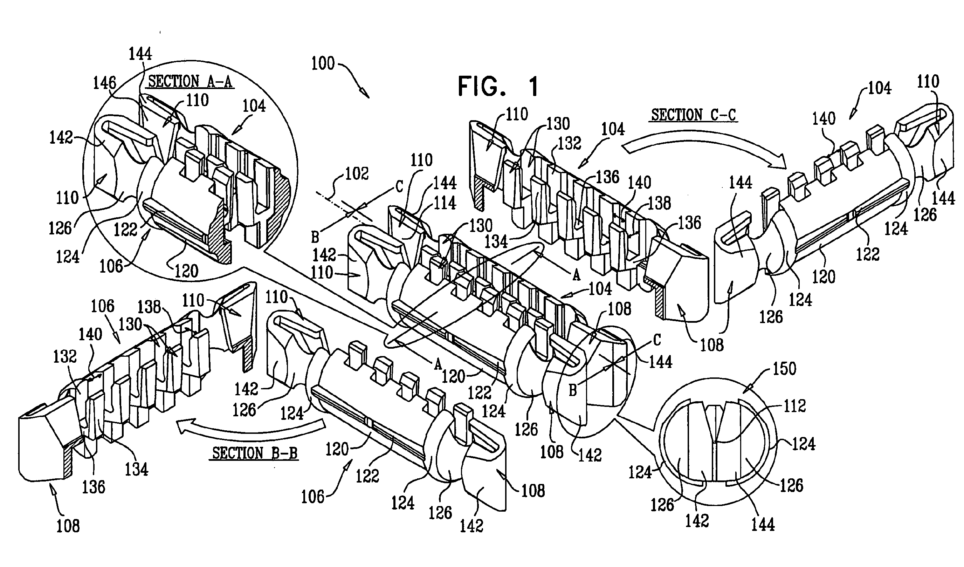

Reference is now made to Fig. 1, which is a simplified composite, partially pictorial and partially sectional illustration of a device for manual manipulation of a guidewire in medical applications constructed and operative in accordance with a preferred embodiment of the present invention.

As seen in Fig. 1, there is provided a device 100 for manual manipulation of a guidewire (not shown). The device 100 is preferably integrally formed of plastic or any other suitable elastic material, has a generally cylindrical overall configuration and extends along a longitudinal axis 102. The device includes first and second tooth-bearing side portions, here respectively designated by reference numerals 104 and 106, which are joined at their respective ends by end portions 108 and 110 each of which defines a notch. The notches of portions 108 and 110 are designated by respective reference numerals 112 and 114 and their fulcrums lie along or near axis 102.

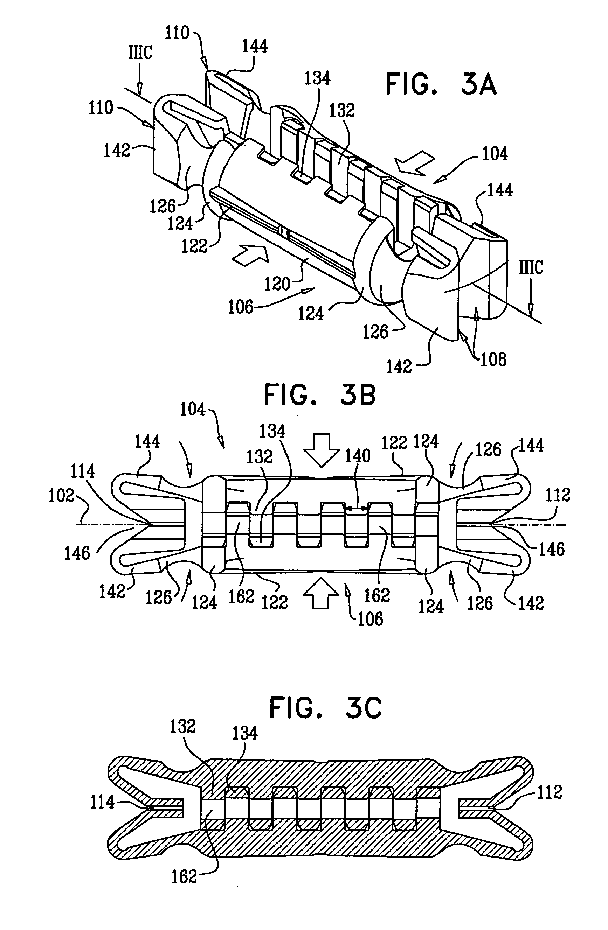

Side portions 104 and 106 may be generally considered to be linearly offset mirror images of each other and are arranged such that when manually pushed together in a direction perpendicular to longitudinal axis 102, as in the compressed operative orientation illustrated in Figs. 3A - 3C, their respective teeth are interdigitated and the device has an overall generally circular cross section.

Each of side portions 104 and 106 includes an outwardly facing back surface 120 having an elongate rib 122 and a pair of transverse end ribs 124. Longitudinally outwardly from each of transverse end ribs 124 there is provided a relatively flexible portion 126 which couples each side portion to end portions 108 and 110.

Each of side portions 104 and 106 also includes on an inwardly facing side thereof a plurality of mutually spaced teeth 130. Each tooth includes a back portion 132 and a forward portion 134 which are joined by a transverse bottom portion 136. Back portion 132 and forward portion 134 extend generally parallel to each other, generally perpendicularly to axis 102 and are spaced from each other by a transverse gap 138.

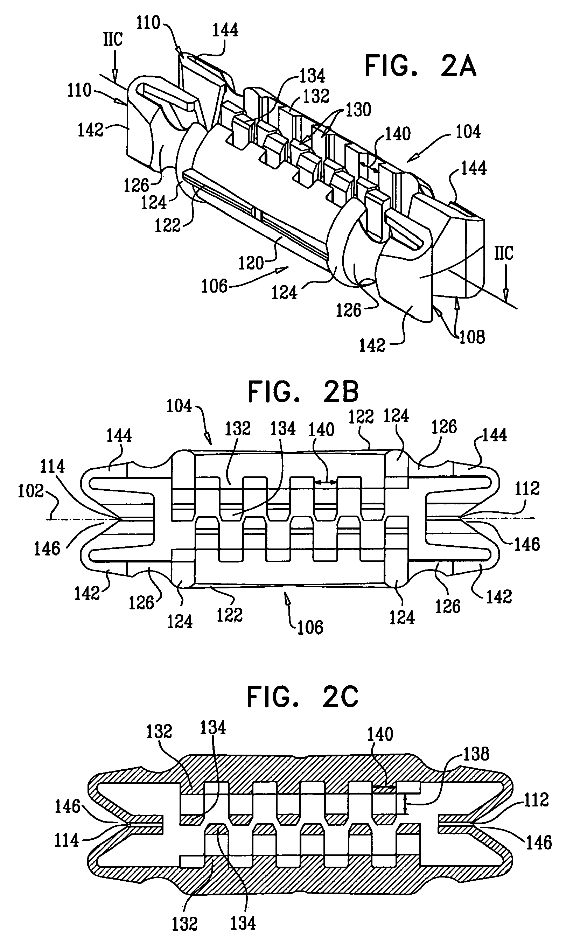

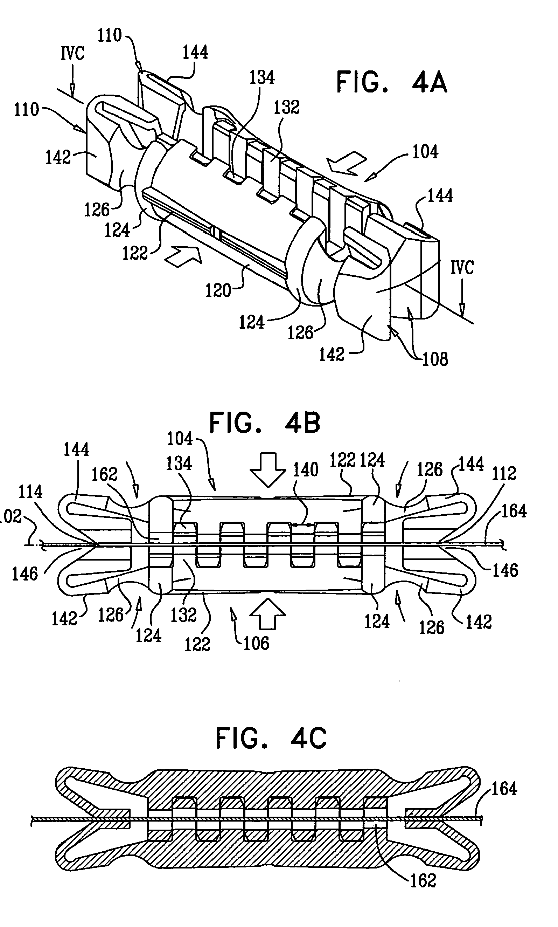

Adjacent teeth 130 on each side portion are spaced by a separation 140, which is preferably equal to the width of each tooth. The teeth 130 of side portion 104 are offset along axis 102 with respect to the teeth 130 of side portion 106 by the width of one tooth, such that when the side portions 104 and 106 are at rest, as seen in Figs. 2A - 2C, the forward portions 134 of the teeth 130 of side portions 104 and 106 are interdigitated generally along axis 102 and when the side portions 104 and 106 are brought into propinquity, as seen in Figs. 3A - 3C and 4A - 4C, the forward portions 134 of teeth 130 are fully or nearly fully seated in separations 140.

End portions 108 and 110 are preferably identical and each includes exterior side portions 142 and 144, which are joined to relatively flexible portions 126 at one end of side portions 104 and 106 and which are bent inwardly to define an axially facing opening 146, having a V-shaped cross section as seen in the plane of Figs. 2B, 3B, 4B and 5B and defining guidewire receiving and positioning notches 112 and 114, whose fulcrums are preferably located along axis 102.

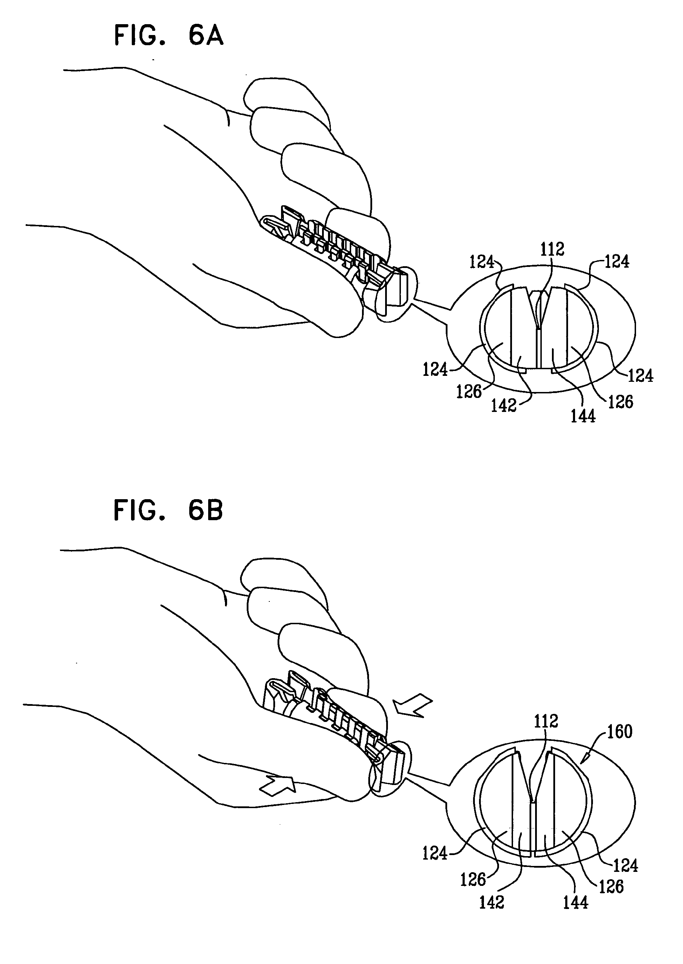

Reference is now made to Figs. 2A, 2B & 2C, which are simplified respective pictorial, top view and sectional illustrations of the device of Fig. 1 in a first, unstressed operative orientation. As seen in Figs. 2A - 2C and as noted above the forward portions 134 of the teeth 130 of side portions 104 and 106 are interdigitated generally along axis 102. It is noted, as seen at enlargement 150 in Fig. 1, that the cross section of the device in its unstressed operative orientation is non-circular. Fig. 6A shows a user holding the device of Fig. 1 in its unstressed operative orientation, as shown in Figs. 2A - 2C.

In order to mount the device of Figs. 1 - 2C onto a side of a guidewire, a user, preferably using his fingers, compresses the device as shown in Fig. 6B to have a generally circular cross section, as seen at enlargement 160 in Fig. 6B. It is appreciated that in order to remove a guidewire from the device of Figs. 1 - 2C a user similarly compresses the device as shown in Fig. 6B.

Reference is now made to Figs. 3A, 3B & 3C which are simplified respective pictorial, top view and sectional illustrations of the device of Fig. 1 in the compressed operative orientation shown in Fig. 6B, in which the device has a generally circular cross section. It is clearly seen in Fig. 6B and in Figs. 3A - 3C that teeth 130 are positioned so that the gaps between respective back and forward portions 132 and 134 thereof are all generally aligned to define a slot 162 which is aligned with notches 112 and 114 to receive a guidewire 164 generally along axis 102.

It will be appreciated that other designs of the teeth 130 of side portions 104 and 106 are possible, provided that the teeth 130 may be positioned so as to define the slot 162 when the device is in the compressed operative orientation shown in Fig. 6B.

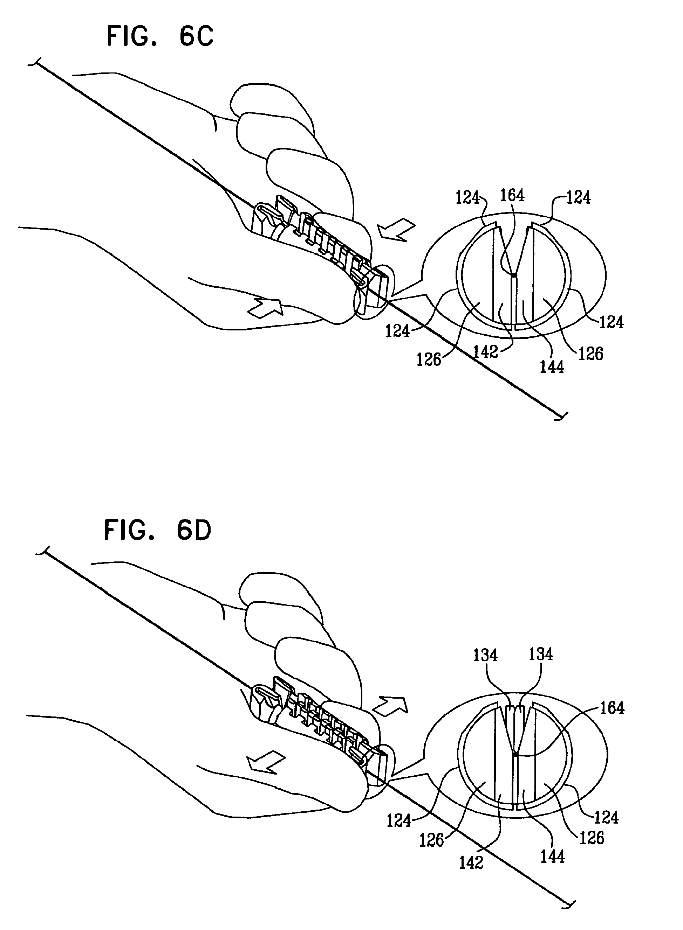

Reference is now made to Figs. 4A, 4B & 4C, which are simplified respective pictorial, top view and sectional illustrations of the device of Fig. 1 in the second, compressed operative orientation in which the device has a generally circular cross section, having guidewire 164 placed, but not retained, therein. This operative state is shown in Fig. 6C.

Once the device has been mounted onto the guidewire such that the guidewire is seated in slot 162 generally along axis 102, the user may relax the compression on the device allowing the device to return to its at rest orientation as shown in Figs. 2A - 2C, but for the presence of the guidewire 164 in the gaps 138 between the back and forward portions 132 and 134 of the teeth 130. This operative state is shown in Fig. 6D. It is appreciated that the guidewire 164 is gripped by the forward portions 134 of the interdigitated teeth 130, such that axial and rotational movement of the device produces corresponding axial and rotational movement of the guidewire 164 about axis 102. It is further appreciated that the guidewire 164 may be axially and rotationally moved by a user about axis 102 without producing corresponding axial and rotational movement of the device.

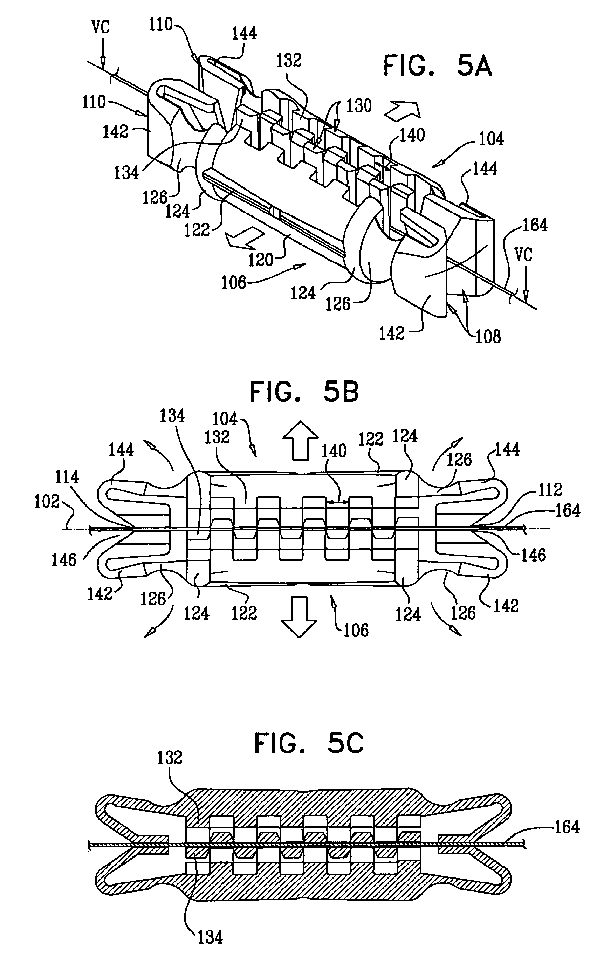

Reference is now made to Figs. 5A, 5B & 5C, which are simplified respective pictorial, top view and sectional illustrations of the device of Fig. 1 in a third, partially compressed, operative orientation, having a guidewire retained therein such that axial and rotational movement of the device produces concomitant axial and rotational movement of the guidewire.

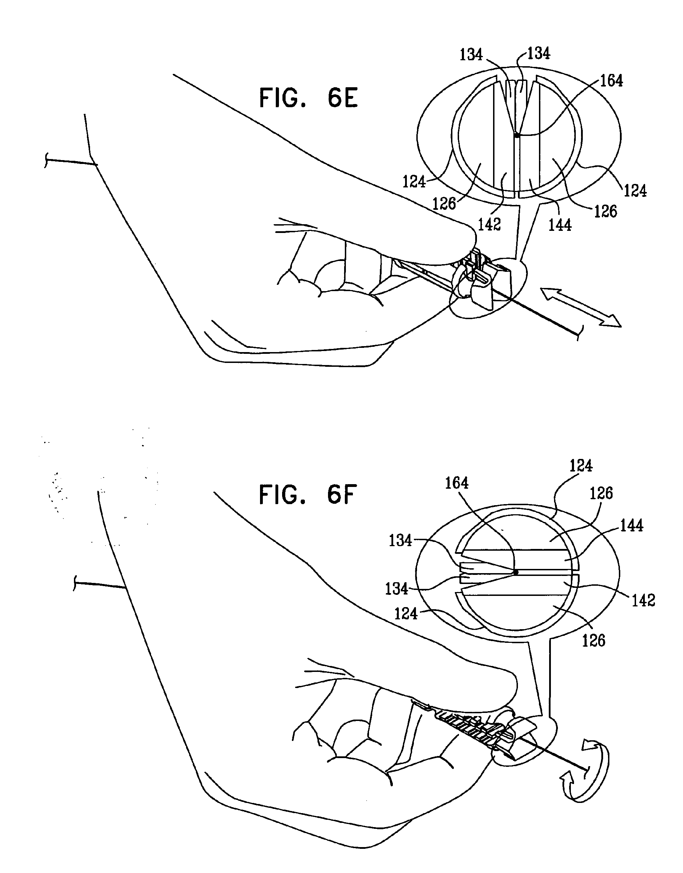

Fig. 6E illustrates manual axial movement of the device of Fig. 1 and the guidewire 164 in the operative orientation shown in Figs. 5A - 5C and Fig. 6F illustrates manual rotation of the device of Fig. 1 and the guidewire 164 in the operative orientation shown in Figs. 5A - 5C through 90 degrees.

It will be appreciated by persons skilled in the art that the present invention is not limited by what has been particularly claimed hereinbelow. Rather the scope of the present invention includes various combinations and subcombinations of the features described hereinabove as well as modifications and variations thereof as would occur to persons skilled in the art upon reading the foregoing description with reference to the drawings and which are not in the prior art. In particular, it will be appreciated that the device may be useful for manual manipulation of any partially rigid wire and is not limited for use with a guidewire only.

## Claims

1. A device for manual manipulation of a guidewire comprising:
a generally cylindrical, integrally formed, compressible element arranged along a longitudinal axis and being compressible transversely to said longitudinal axis and having at least fully compressed and partially compressed operative orientations,
said element in said fully compressed operative orientation defining an axial slot adapted for slidable side mounting of said element onto a guidewire,
said element in said partially compressed operative orientation being adapted for tightly engaging a guidewire in said slot such that axial and rotational movement of said element produces corresponding axial and rotational movement of said guidewire.

2. A device according to claim 1 wherein said element includes first and second tooth bearing side portions each having a plurality of mutually spaced teeth, said mutually spaced teeth each having forward and back portions separated by a gap.

3. A device according to claim 2 wherein first and second end portions are integrally formed with said first and second tooth-bearing side portions, said first and second end portions defining respective first and second notches which are mutually aligned along said longitudinal axis.

4. A device according to claim 3, wherein said first and second tooth-bearing side portions are arranged so as to be:
displaceable towards each other in directions transverse to said longitudinal axis such that said element assumes said fully compressed operative orientation wherein said teeth of said first and second tooth-bearing side portions are interdigitated such that said gaps of said plurality of mutually spaced teeth on both of said first and second tooth-bearing side portions are aligned along said longitudinal axis to define said slot extending between said first and second notches along said longitudinal axis;
displaceable away from each other in directions opposite to said directions transverse to said longitudinal axis when a guidewire is located in said slot, such that said element assumes said partially compressed operative orientation wherein said teeth of said first and second tooth-bearing side portions grip said guidewire along said longitudinal axis, whereby axial and rotational movement of said element is effective to produce corresponding axial and rotational movement of said guidewire.

5. A device according to claim 3 or 4 wherein said first and second tooth-bearing side portions each include an outwardly facing back surface having an elongate rib and a pair of transverse end ribs and relatively flexible portions extending longitudinally outwards from each of said transverse end ribs and coupling said first and second tooth-bearing side portions to said first and second end portions.

6. A device according to claim 5 wherein said first and second end portions each include two exterior side portions joined to said relatively flexible portions and being bent inwardly to define an axially facing opening having a v-shaped cross section.

7. A device according to any of claims 3 - 6 wherein said first and second end portions are identical.

8. A device according to any of claims 2 - 7 wherein said forward and back portions of said mutually spaced teeth are joined by a transverse bottom portion.

9. A device according to any of claims 2 - 8 wherein said forward and back portions of said mutually spaced teeth extend parallel to each other and perpendicular to said longitudinal axis.

10. A device according to any of claims 2 - 9 wherein adjacent ones of said mutually spaced teeth are spaced by a separation, said separation being equal to a width of each of said mutually spaced teeth.

11. A device according to claim 10 wherein said mutually spaced teeth of said first tooth-bearing side portion are offset in a direction along said longitudinal axis with respect to said mutually spaced teeth of said second tooth-bearing side portion by said width of each of said mutually spaced teeth, such that when said element is in said uncompressed orientation said forward portions of said mutually spaced teeth of said first and second tooth-bearing side portions are interdigitated along said longitudinal axis and when said element assumes said fully compressed operative orientation, said forward portions of said mutually spaced teeth are fully or nearly fully seated in said separations.

12. A device according to any of claims 2 - 11 wherein said first and second tooth-bearing side portions are linearly offset mirror images of each other.

13. A device according to any of the preceding claims wherein said guidewire may be axially and rotationally moved by a user without producing corresponding axial and rotational movement of said element, when said element is in said partially compressed operative orientation.

14. A device according to any of the preceding claims wherein said element assumes an overall circular cross section when said element is in said fully compressed orientation.

15. A device according to any of the preceding claims wherein said element is integrally formed of an elastic material.
